# EUROPEAN PATENT SPECIFICATION

(11) **EP 2 886 085 B1**
(45) Date of publication and mention of the grant of the patent: **15.03.2017**
(21) Application number: 14197339.6
(22) Date of filing: 11.12.2014
(51) Int. Cl.: A61F 2/36, A61F 2/30

(54) **Dual modulus orthopaedic prosthesis stem**
Doppelmodus-Schaft für orthopädische Prothese
Tige de prothèse orthopédique à module double

(30) Priority: 18.12.2013 US 201314132917
(43) Date of publication of application: 24.06.2015
(73) Proprietor: DePuy Synthes Products, LLC, Raynham, MA 02767-0350 (US)
(72) Inventor: Armacost, John M., Warsaw, IN Indiana 46581 (US); Keefer, Ryan C., Warsaw, IN Indiana 46581 (US); McAnelly, Jeffrey A., Warsaw, IN Indiana 46581 (US)
(74) Representative: Belcher, Simon James

(56) References cited:
- WO-A1-93/06793
- WO-A2-02/13730
- DE-A1- 3 442 845
- US-A1- 2008 167 723
- US-A1- 2008 255 675

## Description

The invention relates to orthopaedic prostheses, including prostheses which can be used in hip replacement surgery.

Joint arthroplasty is a well-known surgical procedure by which a diseased and/or damaged natural joint is replaced by a prosthetic joint. The prosthetic joint may include a prosthesis that is implanted into one or more of the patient's bones. Many hip prostheses include a femoral prosthesis that is implanted into a patient's femur. A femoral prosthesis typically includes an elongated stem component that is received in the medullary canal of the patient's femur and a spherically-shaped head component that bears against the patient's acetabulum or a prosthetic replacement acetabular cup.

Many femoral prostheses are formed from metallic materials or a combination of metallic and polymeric materials. According to Wolff's law, a patient's bone tissue will remodel in proportion to the stress applied it. Because elongated stem components formed from metal typically have an elastic modulus greater than the elastic modulus of the patient's bone, metallic stem components may shield the patient's bone from stress such that the proximal femoral bone does not remodel to an effective degree, possibly resulting in a loss of support for the implant and/or implant failures.

WO-A-02/13730 discloses an orthopaedic prosthesis that includes a proximal body including a porous metal body and a metal core. The metal core includes a first end positioned proximal of the sheath and configured to receive a femoral head component, a first core segment positioned in the sheath and a second core segment positioned distal of the sheath.

The invention provides an orthopaedic prosthesis as defined in claim 1.

Optionally, the ratio of the first cross-sectional area to the second cross-sectional area may be between 0.35 and 0.47. Optionally, the ratio of the first cross-sectional area to the second cross-sectional area may be less than 0.56.

Optionally, when the orthopaedic prosthesis is viewed in a second transverse plane extending through the sheath of the metallic foam shell, the sheath may define a third cross-sectional area, the sheath and the first core segment may define a fourth cross-sectional area, and a ratio of the third cross-sectional area to the fourth cross-sectional area may be greater than 0.38. Optionally, the ratio of the third cross-sectional area to the fourth cross-sectional area may be between 0.46 and 0.54.

Optionally, the ratio of the third cross-sectional area to the fourth cross-sectional area may be less than 0.70. Optionally, the ratio of the third cross-sectional area to the fourth cross-sectional area may be greater than the ratio of the first cross-sectional area to the second cross-sectional area.

Optionally, the cover layer of the metallic foam shell may extend distally from a proximal end attached to the sheath, and the transverse plane extends through the proximal end of the cover layer. When the orthopaedic prosthesis is viewed in the transverse plane, the cover layer has a first thickness, the second core segment has a second thickness, and a ratio of the first thickness to the second thickness may be greater than 0.46.

Optionally, the first thickness may be greater than 3 mm. When the orthopaedic prosthesis is viewed in the transverse plane, a medial surface of the first core segment may be convex and a lateral surface of the first core segment may be convex.

Optionally, when the orthopaedic prosthesis is viewed in the transverse plane, the medial surface of the first core segment may be defined by a first radius, and the lateral surface of the first core segment may be defined by a second radius that is greater than the first radius.

Optionally, the first core segment of the metallic core may have a medial surface and a lateral surface positioned opposite the medial surface of the first core segment, and the sheath may have a medial surface and a lateral surface positioned opposite the medial surface of the sheath. When the orthopaedic prosthesis is viewed in the transverse plane, a first thickness may be defined between a lateral-most point of the lateral surface of the sheath and a lateral-most point of the lateral surface of the first core segment, a second thickness may be defined between a medial-most point of the medial surface of the sheath and a medial-most point of the medial surface of the first core segment, and a ratio of the second thickness to the first thickness may be greater than 0.14.

Optionally, the orthopaedic prosthesis includes an elongated femoral stem component having a longitudinal axis. The stem component includes the metallic foam shell including the sheath, and the metallic core including the neck positioned proximal of the sheath that is configured to receive a femoral head component, the first core segment positioned in the sheath, and the second core segment positioned distal of the sheath. The metallic foam shell further includes the cover layer extending distally from the sheath and engaging only the lateral surface of the second core segment. The cover layer defines the first cross-sectional area at a first point on the longitudinal axis. The cover layer and the second core segment define the second cross-sectional area at the first point, and the ratio of the first cross-sectional area to the second cross-sectional area is greater than 0.30.

The invention is described below by way of example with reference to the accompanying drawings, in which:
FIG. 1 is a perspective view of an orthopaedic implant.
FIG. 2 is a perspective view of a core of the orthopaedic implant of FIG. 1.
FIG. 3 is a cross-sectional view of the orthopaedic implant taken along the line 3-3 in FIG. 1.
FIG. 4 is a cross-sectional view of the orthopaedic implant taken along the line 4-4 in FIG. 3.
FIG. 5 is a cross-sectional view of the orthopaedic implant taken along the line 5-5 in FIG. 3.
FIG. 6 is a cross-sectional view of the orthopaedic implant taken along the line 6-6 in FIG. 3.
FIG. 7 is a cross-sectional view of the orthopaedic implant taken along the line 7-7 in FIG. 3.
FIG. 8 is a simplified block diagram of a process for manufacturing the orthopaedic implant of FIGS. 1 to 7.
FIG. 9 is a table showing cross-sectional areas of various components of the orthopaedic implant of FIG. 1 at various points along the length of the implant.

Terms representing anatomical references, such as anterior, posterior, medial, lateral, superior, inferior, transverse, and coronal, may be used throughout this document to refer to the orthopaedic implants or prostheses and surgical instruments described herein as well as to refer to the patient's natural anatomy. Such terms have well-understood meanings in both the study of anatomy and the field of orthopaedics. Use of such anatomical reference terms in this document is intended to be consistent with their well-understood meanings unless noted otherwise.

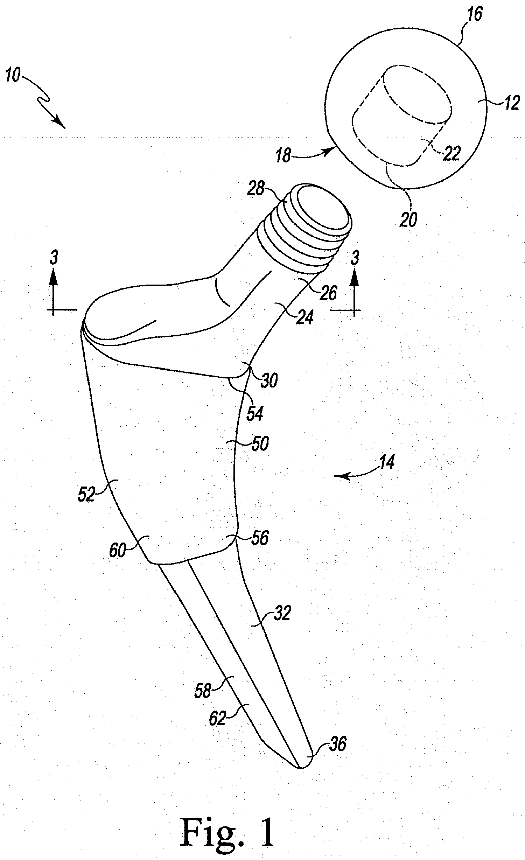

Referring to the drawings, FIG. 1 shows a femoral orthopaedic implant 10 of a hip prosthesis which includes a head component 12 and an elongated stem component 14 that is configured to be inserted into an intramedullary canal of a patient's surgically-prepared femur (not shown). The head component 12 includes a spherical outer surface 16 configured to engage a patient's natural acetabulum (not shown) or a prosthetic acetabular cup implanted into the patient's pelvic bone. The head component 12 also includes a distal surface 18 having an opening 20 defined therein, and an inner wall (not shown) extends inwardly from the opening 20 to define an aperture 22 in the head component 12.

The stem component 14 of the implant 10 includes a core 24 having a neck 26 configured to be coupled to the head component 12. In the device shown in the drawings, the neck 26 includes a plurality of external threads 28 that are configured to engage with a plurality of internal threads (not shown) lining the aperture 22 of the head component 12. The neck and the head component may also be configured to be press fit, taper fit, or secured together by other fastening means.

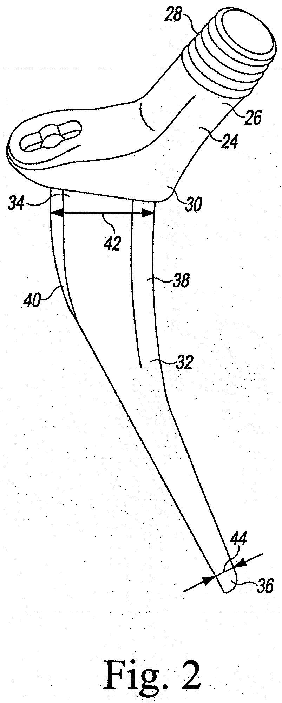

As shown in FIG. 2, the core 24 of the stem component 14 also includes a collar 30 and a core body 32 extending distally from the collar 30. As shown in FIG. 1, the neck 26 extends medially and proximally from the collar 30. In the device shown in the drawings, the neck 26, the collar 30, and the core body 32 are formed as a monolithic structure (e.g., a single moulded or cast part). The components of the core 24 (e.g., the neck 26, the collar 30, and the core body 32) may also be formed as separate components secured to one another by a mechanical fastener (e.g., screw, bolt, taper fit, etc.), adhesive, or other suitable fastener.

The stem core 24 is formed from an implant grade metallic material having a high tensile strength and a high elastic modulus (i.e., a high material stiffness). The term "high tensile strength" is used to refer to a tensile strength that is greater than 650 MPa. The term "high elastic modulus" is used to refer to an elastic modulus or modulus of elasticity that is greater than or equal to 100 GPa. In the device shown in the drawings, the core 24 is formed from cobalt-chromium alloy ("CoCr") having a minimum ultimate tensile strength of 650 MPa and an elastic modulus of approximately 195 GPa. The core 24 may be formed any material having a high tensile strength and a high elastic modulus, including, for example, a titanium alloy such as Ti-6Al-4V, which has a minimum ultimate tensile strength of 750 MPa and an elastic modulus of approximately 105 GPa.

As described above, the core 24 of the stem component 14 includes a core body 32, which lies generally in the coronal plane of a patient's body when the implant 10 is secured to the patient's femur. As shown in FIG. 2, the core body 32 of the core 24 extends from a proximal end 34 attached to the collar 30 to a distal end 36, and the core body 32 has a longitudinal axis 74 that extends through the ends 34, 36. The core body 32 includes a medial surface 38 and a lateral surface 40 positioned opposite the medial surface 38. When the core 24 of the stem component 14 is viewed in the coronal plane, the core body 32 has a thickness 42 at the proximal end 34, which is defined between the surfaces 38, 40 of the core body 32. The core body 32 has another thickness 44 defined between the surfaces 38, 40 at the distal end 36. In the device shown in the drawings, the thickness 44 is less than the thickness 42, and the core body 32 tapers to decrease in thickness between the proximal end 34 and the distal end 36. In the device shown in the drawings, the thickness 42 is equal to approximately 18 mm, and the thickness 44 is equal to approximately 3.5 mm. The thicknesses 42, 44 may be varied depending on the patient's anatomy and the performance requirements of the implant 10.

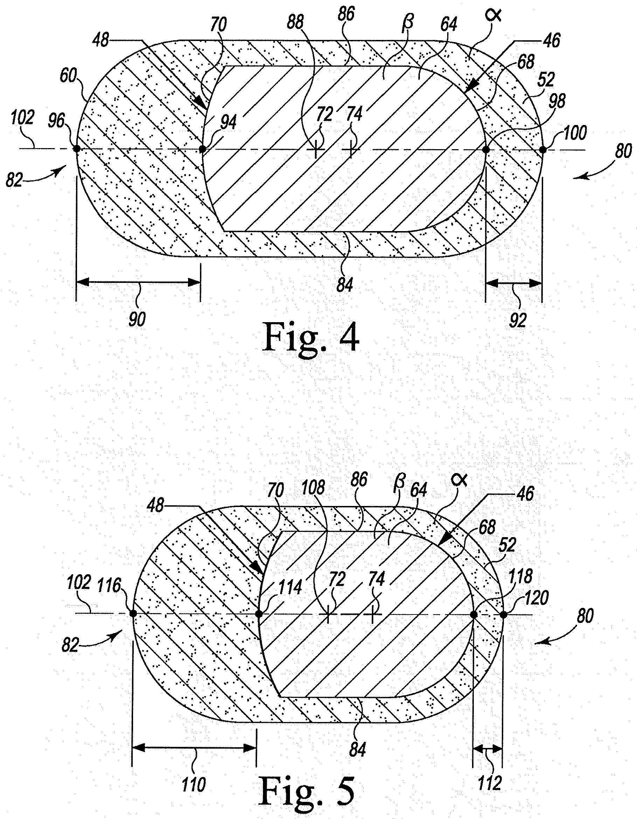

In the device shown in the drawings, the medial surface 38 of the core body 32 is convex. As described below, the medial surface 38 is defined by a radius 46 (see FIGS. 4 to 7) that decreases in magnitude as the medial surface 38 extends from the proximal end 34 of the core body 32 to the distal end 36. The lateral surface 40 is also convex in the device shown in the drawings. The lateral surface 40, like the medial surface 38, is defined by a radius 48 (see FIGS. 4 to 7) that decreases in magnitude as the lateral surface 40 extends from the proximal end 34 of the core body 32 to the distal end 36. While the radii 46, 48 decrease in magnitude, the magnitude of the radius 48 of the lateral surface 40 at each point along the longitudinal axis 74 of the core body 32 is greater than the magnitude of the radius 46 of the medial surface 38.

Returning to FIG. 1, the stem component 14 of the implant 10 also includes a shell 50 that is secured to the core 24. The shell 50 has a sheath 52 that has a proximal end 54 attached to the collar 30 and a distal end 56 positioned between the collar 30 and the distal end 36 of the core 24. The shell 50 also includes a cover layer 58 that extends distally from the sheath 52 to the distal end 36 of the core body 32. In the device shown in the drawings, the sheath 52 and the cover layer 58 are formed as a monolithic structure. The components of the shell 50 (e.g., sheath 52 and the cover layer 58) may instead be formed as separate components. The separate components may be secured to one another by a mechanical fastener (e.g., screw, bolt, taper fit, etc.), adhesive, or other suitable fastener or secured separately to the core 24.

The shell 50 of the stem component 14 is formed from a metallic foam matrix having a low elastic modulus. The term "low elastic modulus" is used to refer to an elastic modulus or modulus of elasticity similar to that of a patient's natural femur (i.e., between 10 GPa and 20 GPa). In the device shown in the drawings, the shell 50 is formed from a foam matrix of titanium having an elastic modulus of approximately 10 GPa and an ultimate tensile strength of the foam matrix of titanium is approximately 35 MPa. In that way, the shell 50 has an elastic modulus that is closer to that of a patient's femur. The shell 50 may be formed any metallic foam matrix having a low elastic modulus, such as, for example, a CoCr foam matrix having an elastic modulus of approximately 19 GPa, a CoCr alloy foam matrix, a titanium foam alloy matrix, or other foam matrix.

As described above, the core 24 of the stem component 14 in the device shown in the drawings is formed from CoCr having an elastic modulus of approximately 195 GPa while the shell 50 is formed from a foam matrix of titanium having an elastic modulus of approximately 10 GPa. The elastic modulus of the shell 50 is therefore approximately 5% of the elastic modulus of the core 24. The core may be formed from CoCr having an elastic modulus of approximately 195 GPa and the shell may be formed from a CoCr foam matrix having an elastic modulus of approximately 19 GPa. The elastic modulus of the shell is then approximately 10% of the elastic modulus of the core. The core may be formed from Ti-6Al-4V having an elastic modulus of approximately 105 GPa and the shell may be formed from a titanium foam matrix having an elastic modulus of approximately 10 GPa. The elastic modulus of the shell is then about 10% of the elastic modulus of the core.

As shown in FIG. 1, the sheath 52 of the shell 50 has an outer surface 60, and the cover layer 58 has an outer surface 62. The outer surfaces 60, 62 define a portion of the external geometry of the implant 10. As such, the outer surfaces 60, 62 engage the portion of the patient's femur defining the intramedullary canal when the implant 10 is inserted into the proximal end of the patient's surgically-prepared femur. In the device shown in the drawings, the outer surface 60 of the sheath 52 is porous to enable bone ingrowth fixation, and the outer surface 62 of the cover layer 58 is non-porous. The cover layer 58 may also be porous.

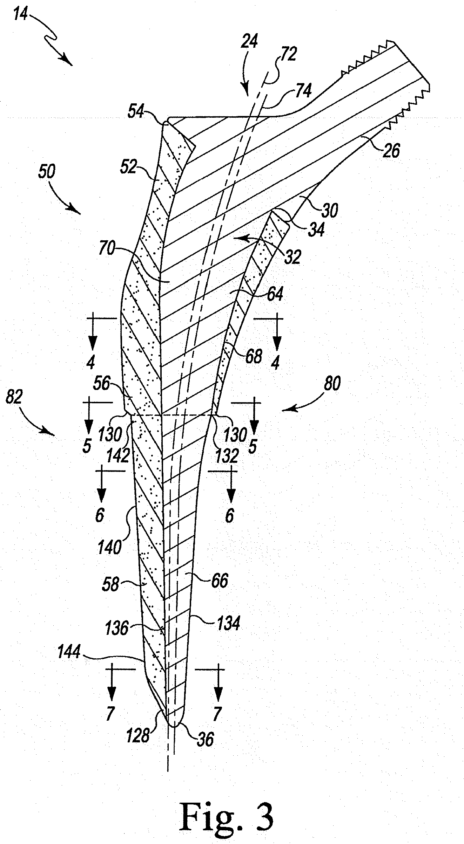

As shown in FIG. 3, the core body 32 of the stem core 24 extends through the sheath 52 of the shell 50. The core body 32 includes a core segment 64 that is positioned in the sheath 52, and a core segment 66 that is positioned distal of the sheath 52. The sheath 52 is attached to and encases a medial surface 68 and a lateral surface 70 of the core segment 64. The surfaces 68, 70 form proximal sections of the medial surface 38 and lateral surface 40, respectively, of the core body 32. In the device shown in the drawings, each of the surfaces 68, 70 extends from an outer anterior surface 84 of the core body 32 to an outer posterior surface 86 of the core body 32.

The core body 32 (i.e., the core segments 64, 66) and the shell 50 (i.e., the sheath 52 and cover layer 58) cooperate to define a longitudinal axis 72 of the stem component 14, which extends between the proximal end 34 and the distal end 36. As described above, the core body 32 has a longitudinal axis 74 that is defined between the ends 34, 36, and the axis 74 is offset from the axis 72. In the device shown in the drawings, the axis 74 is offset in the medial direction from the axis 72 such that the core body 32 is biassed toward the medial side 80 of the stem component 14 and away from the lateral side 82 of the stem component 14. Additionally, the thickness (referred to herein as the "lateral thickness") of the shell 50 on the lateral side 82 of the stem component 14 is greater than the thickness (referred to herein as the "medial thickness") of the shell 50 on the medial side 80 of the stem component 14.

As described above, the sheath 52 of the foam shell 50 has a proximal end 54 and a distal end 56. In the device shown in the drawings, the ratio of the medial thickness of the shell 50 to the lateral thickness of the shell 50 is between 0.80 and 0.17 at each point along the longitudinal axis 72 between the ends 54, 56 of the sheath 52. In other words, at any point between the ends 54, 56 of the sheath 52, the medial thickness may be 17% to 80% of the lateral thickness. Optionally, the ratio at each point may be between 0.95 and 0.14.

For example, as shown in FIG. 4, the sheath 52 has a lateral thickness 90 and a medial thickness 92 when viewed in a transverse plane extending through the stem component 14 at a point 88 on the axis 72 between the proximal end 54 and the distal end 56 of the sheath 52. The lateral thickness 90 of the sheath 52 is defined between a lateral-most point 94 of the lateral surface 70 of the core segment 64 and a lateral-most point 96 of the outer surface 60 of the sheath 52. The medial thickness 92 of the sheath 52 is defined between a medial-most point 98 of the medial surface 68 of the core segment 64 and a medial-most point 100 of the outer surface 60 of the sheath 52. Each of the points 94, 96, 98, 100 lies in the coronal plane, as indicated by an imaginary line 102.

As shown in FIG. 4, the lateral thickness 90 is greater than the medial thickness 92 at the point 88 on the longitudinal axis 72. In other words, the thickness 90 of the shell 50 on the lateral side 82 of the stem component 14 is greater than the thickness 92 of the shell 50 on the medial side 80 of the stem component 14. In the device shown in the drawings, the lateral thickness 90 is greater than 5 mm, and the medial thickness 92 is between 2.0 and 4.5 mm. The ratio of the medial thickness 92 to the lateral thickness 90 is less than or equal to 0.40 at the point 88. Optionally, the ratio of the medial thickness 92 to the lateral thickness 90 can be about 0.37 at the point 88.

As shown in FIGS. 3 to 5, the thickness of the sheath 52 of the shell 50 on the medial side 80 of the stem component 14 generally decreases from the proximal end 54 and the distal end 56 of the sheath 52. As shown in FIG. 5, the sheath 52 has a lateral thickness 110 and a medial thickness 112 when viewed in a transverse plane extending through the stem component 14 at a point 108 on the longitudinal axis 72 at the distal end 56 of the sheath 52. The lateral thickness 110 of the sheath 52 is defined between a lateral-most point 114 of the lateral surface 70 of the core segment 64 and a lateral-most point 116 of the outer surface 60 of the sheath 52. The medial thickness 112 of the sheath 52 is defined between a medial-most point 118 of the medial surface 68 of the core segment 64 and a lateral-most point 120 of the outer surface 60 of the sheath 52. Each of the points 114, 116, 118, 120 lies in the coronal plane, as indicated by the imaginary line 102.

As shown in FIG. 5, the lateral thickness 110 of the sheath 52 is greater than the medial thickness 112 of the sheath 52 at the point 108 on the longitudinal axis 72. In the device shown in the drawings, the lateral thickness 110 is greater than 5 mm. Additionally, the medial thickness 112 at the distal end 56 of the sheath 52 is less than the medial thickness 92 (see FIG. 4). As described above, the medial thickness 92 of sheath 52 in the device shown in the drawings is between 2.0 and 4.5 mm, and the medial thickness 112 of the sheath 52 is between 1.0 and 1.5 mm. The ratio of the medial thickness 112 to the lateral thickness 110 is less than or equal to 0.30 at the point 108. Optionally, the ratio of the medial thickness 112 to the lateral thickness 110 may be about 0.17 at the point 108.

Returning to FIG. 3, the outer surface 60 of the sheath 52 has a curved or rounded distal surface section 130 at the distal end 56 of the sheath 52. The distal surface section 130 has an edge 132 that extends around the core body 32 of the stem core 24 and the cover layer 58 of the shell 50. As described above, the core body 32 also includes a core segment 66 that is positioned distal of the end 56 of the sheath 52. The core segment 66 extends from the edge 132 of the sheath 52 to the distal end 36 of the core body 32.

The core segment 66 has a medial surface 134 that forms a distal section of the medial surface 38 of the core body 32. The core segment 66 also has a lateral surface 136 that forms a distal section of the lateral surface 40 of the core body 32. The medial surface 134 and the lateral surface 136 extend from the outer anterior surface 84 of the core body 32 to the outer posterior surface 86 of the core body 32. As shown in FIG. 3, the cover layer 58 of the shell 50 is attached to only the lateral surface 136 of the core segment 66. The medial surface 134 of the core segment 66 forms a portion of the external geometry of the implant 10 such that the medial surface 134 may engage the patient's bone when the implant 10 is inserted into the intramedullary canal.

As shown in FIG. 3, the cover layer 58 has a body 140 that extends from a proximal end 142 attached to the sheath 52. The cover layer body 140 extends to a distal end 144 connected to a tip 128 of the cover layer 58. The tip 128 extends to the distal end of the stem component 14. In the device shown in the drawings, the tip 128 is shaped to provide relief during insertion of the stem component 14 into the proximal end of the patient's femur. The tip 128 may be omitted and the body 140 of the cover layer 58 may extend to the distal end of the stem component 14.

The stem component 14 has a medial-lateral thickness that is the sum of the thickness of the cover layer body 140 and the thickness of the lower core segment 66. In the device shown in the drawings, the ratio of the thickness of the cover layer body 140 to the thickness of the segment 66 is between 0.80 and 0.57 at each point along the longitudinal axis 72 between the ends 142, 144 of the cover layer body 140. In other words, at any point between the ends 142, 144 of the body 140, the cover layer body thickness may be 57% to 80% of the thickness of the core segment 66. Optionally, the ratio at each point may be between 0.96 and 0.46. As shown in FIG. 3, the tip 128 of the cover layer 58 is tapered such that the thickness of the cover layer 58 decreases from the distal end 144 of the cover layer body 140 to the distal end of the stem component 14.

As shown in FIGS. 3, 6 and 7, the cover layer 58 makes up a significant percentage of the overall thickness of stem component 14. The ratio of the thickness of the cover layer body 140 to the overall thickness of the stem component 14 is between 0.45 and 0.36 at each point between the ends 142, 144 of the cover layer body 140. In other words, the cover layer body 140 may be 36% to 45% of the overall thickness of the stem component 14 between the ends 142, 144. Optionally, the ratio of the thickness of the cover layer body 140 to the overall thickness may be between 0.54 and 0.29.

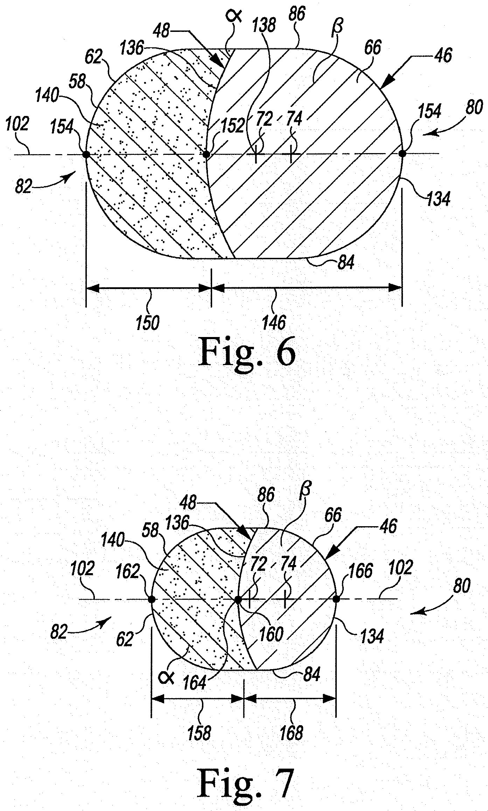

In the device shown in the drawings, the thickness of the cover layer body 140 decreases along the longitudinal axis 72 between the ends 142, 144. For example, as shown in FIG. 6, the cover layer body 140 has a lateral thickness 150 when viewed in a transverse plane that extends through the stem component 14 between the ends 142, 144 at the point 138 on the axis 72. The lateral thickness 150 of the cover layer body 140 is defined between a lateral-most point 152 of the lateral surface 136 of the core segment 66 and a lateral-most point 154 of the outer surface 62 of the cover layer 58. As shown in FIG. 6, the lateral thickness 150 is greater than 4.5 mm.

As shown in FIG. 6, the core segment 66 at the point 138 has a thickness 146 defined between the lateral-most point 152 of the core segment 66 and a medial-most point 148 of the core segment 66. The thickness 146 in the device shown in the drawings is greater than 7.0 mm. The ratio of the lateral thickness 150 to the core thickness 146 at the point 138 is between 0.65 and 0.57. Optionally, the ratio may be about 0.59 at the point 138. Additionally, at the point 138, the ratio of the lateral thickness 150 to the overall thickness of the stem component 14 may be between 0.39 and 0.29. Optionally, the ratio of the lateral thickness 150 to the overall thickness may be about 0.37 at the point 138.

As shown in FIG. 7, the cover layer body 140 has a lateral thickness 158 when viewed in a transverse plane extending through the stem component 14 at a point 164 on the axis 72 at the distal end 144 of the cover layer 58. The lateral thickness 158 of the body 140 is defined between a lateral-most point 160 of the lateral surface 136 of the core segment 66 and a lateral-most point 162 of the outer surface 62 of the cover layer 58. In the device shown in the drawings, the lateral thickness 158 of the body 140 is between 3 mm and 4 mm. In other words, the lateral thickness 158 of the cover layer body 140 at the distal end 144 of the cover layer 58 is less than the lateral thickness 150 of the body 140 between the ends 142, 144.

The core segment 66 at the point 164 has a thickness 166 defined between the lateral-most point 160 of the core segment 66 and a medial-most point 168 of the core segment 66. The thickness 166 in the device shown in the drawings is greater than 3.5 mm. The ratio of the lateral thickness 158 to the core thickness 166 at the point 164 is between 0.85 and 0.57. Optionally, the ratio is about 0.80 at the point 164 along the longitudinal axis 72. Additionally, at the point 164, the ratio of the lateral thickness 158 to the overall thickness of the stem component 14 is between 0.40 and 0.29. Optionally, the ratio of the lateral thickness 158 to the overall thickness is about 0.45 at the point 164.

As shown in FIGS. 3 to 7, the foam shell 50 comprises a significant portion of the stem component 14, as indicated by the ratio between the cross-sectional area of the foam shell 50 (identified as α in FIGS. 4 to 7) to the total cross-sectional area of the stem component 14 (designated τ in FIGS. 4 to 7). It should be appreciated that in the device shown in the drawings the total cross-sectional area of the stem component comprises the cross-sectional area of the foam shell 50 and the cross-sectional area of the core body 32. In the device shown in the drawings, the ratio of the cross-sectional area of the foam shell 50 (designated α in FIGS. 4 to 7) to the cross-sectional area of the stem component 14 (designated β in FIGS. 4 to 7) is between 0.55 and 0.37 at each point along the longitudinal axis 72 of stem component 14 above the tip 128 of the foam shell 50. In other words, the foam shell 50 may form 37% to 55% of the cross-sectional area of the stem component 14 at each of those points. Optionally, the ratio at each point may be between 0.70 and 0.30.

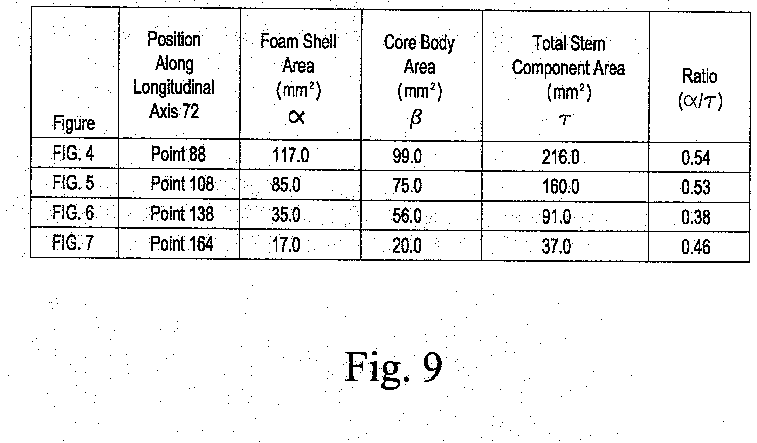

As shown in FIG. 9, a table 170 shows values for the cross-sectional area α of the foam shell 50, the cross-sectional area β of the core body 32, and the cross-sectional area τ of the stem component 14 at each of the points 88, 108, 138, 164 along the axis 72. In the device shown in the drawings, the total cross-sectional area τ of the stem component 14 is equal to the sum of the cross-sectional area α of the foam shell 50 and the cross-sectional area β of the core body 32 at each point along the axis 72 above the tip 128 of the foam shell 50. As shown in the table 170, the ratio of cross-sectional area α to the cross-sectional area τ ranges from 0.46 to 0.54. Between the ends 54, 56 of the foam sheath 52, the ratio of cross-sectional area α to the cross-sectional area τ is between 0.55 and 0.47. Optionally, the ratio of cross-sectional area α to the cross-sectional area β between the ends 54, 56 of the sheath 52 may be between 0.70 and 0.38.

Between the ends 142, 144 of the cover layer body 140, the ratio of cross-sectional area α to the cross-sectional area β is between 0.47 and 0.37. Optionally, the ratio of cross-sectional area α to the cross-sectional area τ between the ends 142, 144 of the cover layer body 140 may be between 0.56 and 0.30.

In use, the implant 10 is inserted into a proximal end of a patient's surgically-prepared femur. The elongated stem component 14 is received in the intramedullary canal and the sheath 52 and the cover layer 58 of the shell 50 engage the portion of the patient's femur surrounding the canal. The core 24 is sized and shaped to meet the minimum strength requirements of the implant 10, while the shell 50 is configured to possess the external geometry necessary to fit into the intramedullary canal. The combination of the high tensile strength/high elastic modulus core 24 with the low modulus shell 50 results in a reduced stiffness for the implant 10 such that stress shielding of the patient's bone is reduced.

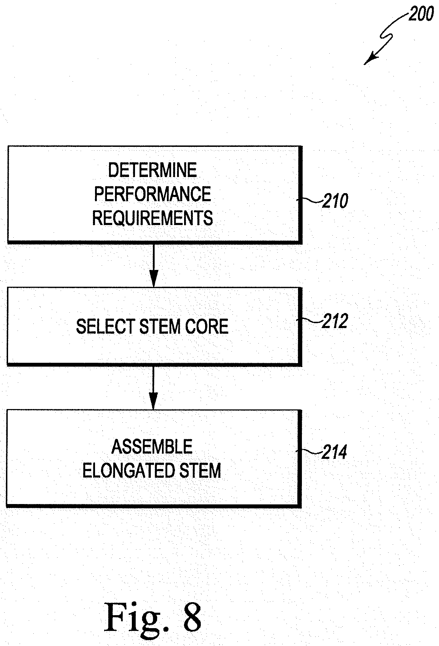

FIG. 8 shows steps in a method 200 of manufacturing the elongated stem component 14 of the implant 10, in which the performance requirements of the stem component 14 are determined and the stem core 24 of the stem component 14 is procured. The stem core 24 and the shell 50 may then be assembled to form the stem component 14.

In block 210, the performance requirements of the stem component 14 are determined. The performance requirements for the stem component 14 may vary between different patients such that a customized stem component 14 may be required. The performance requirements include the external geometry and the minimum endurance and performance requirements, such as, for example, the minimum tensile strength and the minimum stiffness of the stem component 14.

The core 24 of the stem component 14 is selected in block 212. The core 24 may be a generic core that may be used with multiple implant sizes. The core 24 may be a customized, patient-specific component designed to satisfy the performance requirements of a particular patient. Whether the core 24 is generic or custom, the selected core 24 of the component 14 is sized and shaped to provide the minimum strength of the implant 10. The minimum strength of the core 24 is determined in accordance with International Organization for Standardization Standard No. 7206-4:2010 "Implants for Surgery -- Partial and Total Hip Joint Prostheses -- Part 4: Determination of Endurance Properties and Performance of Stemmed Femoral Components" and Standard No. 7206-6:1992 "Implants for Surgery -- Partial and Total Hip Joint Prostheses -- Part 6: Determination of Endurance Properties of Head and Neck Region of Stemmed Femoral Components".

In block 214, the stem component 14 is assembled. To do so, a metallic foam matrix, which will form the shell 50 of the stem component 14, is procured. The metallic foam matrix may be compressed around the core 24 such that the core 24 is received in a channel within the foam matrix. The foam matrix may then be machined to the required external geometry of the shell 50. Alternatively, the metallic foam matrix may be compressed separately into the shape of shell 50. After the matrix is compressed, a channel sized to receive the core 24 may be machined in the shell 50 before the shell 50 is assembled with the core 24.

A sintering operation may be used to secure the shell 50 to the core 24. It should also be appreciated that the shell 50 and the core 24 may be secured by a brazing operation, a press-fit, or other securing means.

## Claims

1. An orthopaedic prosthesis (10), comprising:
a metallic foam shell (50) including a sheath (52), and
a metallic core (24) including (i) a neck (26) positioned proximal of the sheath, the neck being configured to receive a femoral head component, (ii) a first core segment (64) positioned in the sheath, and (iii) a second core segment (66) positioned distal of the sheath,
**characterised in that**
the metallic foam shell further includes a cover layer (58) extending distally from the sheath, the cover layer engaging only a lateral surface (136) of the second core segment,
in which when the orthopaedic prosthesis is viewed in a transverse plane extending through the cover layer of the metallic foam shell, (i) the cover layer defines a first cross-sectional area, (ii) the cover layer and the second core segment define a second cross-sectional area, and (iii) a ratio of the first cross-sectional area to the second cross-sectional area is greater than 0.30.

2. The orthopaedic prosthesis (10) of claim 1, in which the ratio of the first cross-sectional area to the second cross-sectional area is less than 0.56, and preferably is between 0.35 and 0.47.

3. The orthopaedic prosthesis (10) of claim 1, in which when the orthopaedic prosthesis is viewed in a second transverse plane extending through the sheath (52) of the metallic foam shell (50),
the sheath defines a third cross-sectional area,
the sheath and the first core segment define a fourth cross-sectional area, and
a ratio of the third cross-sectional area to the fourth cross-sectional area is greater than 0.38.

4. The orthopaedic prosthesis (10) of claim 3, in which the ratio of the third cross-sectional area to the fourth cross-sectional area is less than 0.70, and preferably is between 0.46 and 0.54.

5. The orthopaedic prosthesis (10) of claim 3, in which the ratio of the third cross-sectional area to the fourth cross-sectional area is greater than the ratio of the first cross-sectional area to the second cross-sectional area.

6. The orthopaedic prosthesis (10) of claim 1, in which:
the cover layer (58) of the metallic foam shell (50) extends distally from a proximal end (142) attached to the sheath (52), and
the transverse plane extends through the proximal end (142) of the cover layer, and when the orthopaedic prosthesis is viewed in the transverse plane: (i) the cover layer has a first thickness, (ii) the second core segment (66) has a second thickness, and (iii) a ratio of the first thickness to the second thickness is greater than 0.46.

7. The orthopaedic prosthesis (10) of claim 6, in which the first thickness is greater than 3 mm.

8. The orthopaedic prosthesis (10) of claim 1, in which when the orthopaedic prosthesis is viewed in the transverse plane, a medial surface (38) of the first core segment (64) is convex and a lateral surface (40) of the first core segment is convex.

9. The orthopaedic prosthesis (10) of claim 8, in which when the orthopaedic prosthesis is viewed in the transverse plane, the medial surface (38) of the first core segment (64) is defined by a first radius, and the lateral surface (40) of the first core segment (64) is defined by a second radius that is greater than the first radius.

10. The orthopaedic prosthesis (10) of claim 1, in which:
the first core segment (64) of the metallic core (24) has a medial surface (38) and a lateral surface (40) positioned opposite the medial surface of the first core segment,
the sheath (52) has a medial surface and a lateral surface positioned opposite the medial surface of the sheath, and
when the orthopaedic prosthesis is viewed in the transverse plane, a first thickness (90) is defined between a lateral-most point (96) of the lateral surface of the sheath (52) and a lateral-most point (94) of the lateral surface (70) of the first core segment (64), (ii) a second thickness (92) is defined between a medial-most point (100) of the medial surface (68) of the sheath (52) and a medial-most point (100) of the medial surface (68) of the first core (64) and (iii) a ratio of the second thickness to the first thickness is greater than 0.14.

11. The orthopaedic prosthesis (10) of claim 1, in which the sheath (52) and the cover layer (58) of the metallic foam shell (50) have a first elastic modulus and the first core segment (64) and the second core segment (66) of the metallic core (32) have a second elastic modulus, the first elastic modulus being approximately one-twentieth of the second elastic modulus.

12. The orthopaedic prosthesis (10) of claim 1, comprising:
an elongated femoral stem component (14) having a longitudinal axis, the stem component comprising:
the metallic foam shell (50) including the sheath (52), and
the metallic core (24) including (i) the neck (26) positioned proximal of the sheath, the neck being configured to receive a femoral head component, (ii) the first core segment (64) positioned in the sheath, and (iii) the second core segment (66) positioned distal of the sheath,
in which (i) the metallic foam shell further includes the cover layer (58) extending distally from the sheath, the cover layer engaging only the lateral surface (136) of the second core segment and defining the first cross-sectional area at a first point on the longitudinal axis, (ii) the cover layer and the second core segment defining the second cross-sectional area at the first point, and (iii) the ratio of the first cross-sectional area to the second cross-sectional area is greater than 0.30.

## Patentansprüche

1. Orthopädische Prothese (10), umfassend:
eine Metallschaumhülle (50), die einen Mantel (52) enthält, und
einen metallischen Kern (24), der (i) einen Hals (26), der proximal zu dem Mantel positioniert ist, wobei der Hals konfiguriert ist, eine Femurkopfkomponente aufzunehmen, (ii) ein erstes Kernsegment (64), das in dem Mantel positioniert ist, und (iii) ein zweites Kernsegment (66) enthält, das distal zu dem Mantel positioniert ist,
**dadurch gekennzeichnet, dass**
die Metallschaumhülle ferner eine Abdeckschicht (58) enthält, die sich distal von dem Mantel erstreckt, wobei die Abdeckschicht nur in eine laterale Fläche bzw. Oberfläche (136) des zweiten Kernsegments eingrifft,
wobei, wenn die orthopädische Prothese in einer Querebene betrachtet wird, die sich durch die Abdeckschicht der Metallschaumhülle erstreckt, (i) die Abdeckschicht einen ersten Querschnittsbereich definiert, (ii) die Abdeckschicht und das zweite Kernsegment einen zweiten Querschnittsbereich definieren, und (iii) ein Verhältnis des ersten Querschnittsbereichs zu dem zweiten Querschnittsbereich größer als 0,30 ist.

2. Orthopädische Prothese (10) nach Anspruch 1, wobei das Verhältnis des ersten Querschnittsbereichs zu dem zweiten Querschnittsbereich kleiner als 0,56 ist, und vorzugsweise zwischen 0,35 und 0,47 ist.

3. Orthopädische Prothese (10) nach Anspruch 1, wobei, wenn die orthopädische Prothese in einer zweiten Querebene betrachtet wird, die sich durch den Mantel (52) der Metallschaumhülle (50) erstreckt,
der Mantel einen dritten Querschnittsbereich definiert,
der Mantel und das erste Kernsegment einen vierten Querschnittsbereich definieren, und
ein Verhältnis des dritten Querschnittsbereichs zu dem vierten Querschnittsbereich größer als 0,30 ist.

4. Orthopädische Prothese (10) nach Anspruch 3, wobei das Verhältnis des dritten Querschnittsbereichs zu dem vierten Querschnittsbereich kleiner als 0,70 ist, und vorzugsweise zwischen 0,46 und 0,54 ist.

5. Orthopädische Prothese (10) nach Anspruch 3, wobei das Verhältnis des dritten Querschnittsbereichs zu dem vierten Querschnittsbereich größer ist als das Verhältnis des ersten Querschnittsbereichs zu dem zweiten Querschnittsbereich.

6. Orthopädische Prothese (10) nach Anspruch 1, wobei:
sich die Abdeckschicht (58) der Metallschaumhülle (50) distal von einem proximalen Ende (142) erstreckt, das an dem Mantel (52) angebracht ist, und
sich die Querebene durch das proximale Ende (142) der Abdeckschicht erstreckt, und, wenn die orthopädische Prothese in der Querebene betrachtet wird: (i) die Abdeckschicht eine erste Dicke aufweist, (ii) das zweite Kernsegment (66) eine zweite Dicke aufweist, und (iii) ein Verhältnis der ersten Dicke zu der zweiten Dicke größer als 0,46 ist.

7. Orthopädische Prothese (10) nach Anspruch 6, wobei die erste Dicke größer als 3 mm ist.

8. Orthopädische Prothese (10) nach Anspruch 1, wobei, wenn die orthopädische Prothese in der Querebene betrachtet wird, eine mediale bzw. mittlere Oberfläche (38) des ersten Kernsegments (64) konvex ist und eine laterale Oberfläche (40) des ersten Kernsegments konvex ist.

9. Orthopädische Prothese (10) nach Anspruch 8, wobei, wenn die orthopädische Prothese in der Querebene betrachtet wird, die mittlere Oberfläche (38) des ersten Kernsegments (64) durch einen ersten Radius definiert ist und die laterale Oberfläche (40) des ersten Kernsegments (64) durch einen zweiten Radius definiert ist, der größer ist als der erste Radius.

10. Orthopädische Prothese (10) nach Anspruch 1, wobei:
das erste Kernsegment (64) des metallischen Kerns (24) eine mediale bzw. mittlere Oberfläche (38) und eine laterale Oberfläche (40) aufweist, die gegenüberliegend bzw. entgegengesetzt zu der mittleren Oberfläche des ersten Kernsegments positioniert ist,
der Mantel (52) eine mediale bzw. mittlere Oberfläche und eine laterale Oberfläche aufweist, die gegenüberliegend bzw. entgegengesetzt zu der mittleren Oberfläche des Mantels positioniert ist, und
wenn die orthopädische Prothese in der Querebene betrachtet wird, eine erste Dicke (90) zwischen einem lateralsten Punkt (96) der lateralen Oberfläche des Mantels (52) und einem lateralsten Punkt (94) der lateralen Oberfläche (70) des ersten Kernsegments (64) definiert ist; (ii) eine zweite Dicke (92) zwischen einem medialsten bzw. mittlersten Punkt (100) der mittleren Oberfläche (68) des Mantels (52) und einem medialsten bzw. mittlersten Punkt (100) der mittleren Oberfläche (68) des ersten Kerns (64) definiert ist, und (iii) ein Verhältnis der zweiten Dicke zu der ersten Dicke größer als 0,14 ist.

11. Orthopädische Prothese (10) nach Anspruch 1, wobei der Mantel (52) und die Abdeckschicht (58) der Metallschaumhülle (50) ein erstes Elastizitätsmodul aufweisen und das erste Kernsegment (64) und das zweite Kernsegment (66) des metallischen Kerns (32) ein zweites Elastizitätsmodul aufweisen, wobei das erste Elastizitätsmodul näherungsweise ein Zwanzigstel des zweiten Elastizitätsmoduls ist.

12. Orthopädische Prothese (10) nach Anspruch 1, umfassend:
eine längliche Femurschaftkomponente (14) mit einer Längsachse, wobei die Schaftkomponente umfasst:
die Metallschaumhülle (50), die den Mantel (52) enthält, und
den metallischen Kern (24), der (i) den Hals (26), der proximal zu dem Mantel positioniert ist, wobei der Hals konfiguriert ist, eine Femurkopfkomponente aufzunehmen, (ii) das erste Kernsegment (64), das in dem Mantel positioniert ist, und (iii) das zweite Kernsegment (66) enthält, das distal zu dem Mantel positioniert ist,
wobei (i) die Metallschaumhülle ferner die Abdeckschicht (58) enthält, die sich distal von dem Mantel erstreckt, wobei die Abdeckschicht nur in die laterale Oberfläche (136) des zweiten Kernsegments eingrifft und den ersten Querschnittsbereich an einem ersten Punkt auf der Längsachse definiert, (ii) die Abdeckschicht und das zweite Kernsegment den zweiten Querschnittsbereich an dem ersten Punkt definieren, und (iii) das Verhältnis des ersten Querschnittsbereichs zu dem zweiten Querschnittsbereich größer als 0,30 ist.

## Revendications

1. Prothèse orthopédique (10), comprenant :
une coque en mousse métallique (50) incluant une gaine (52), et
une âme métallique (24) incluant (i) un col (26) positionné à proximité de la gaine, le col étant configuré pour recevoir un composant de tête fémorale, (ii) un premier segment d'âme (64) positionné dans la gaine, et (iii) un second segment d'âme (66) positionné distal de la gaine,
**caractérisé en ce que**
la coque en mousse métallique inclut en outre une couche couvrante (58) s'étendant de façon distale à partir de la gaine, la couche couvrante engageant uniquement une surface latérale (136) du second segment d'âme,
dans laquelle lorsque la prothèse orthopédique est vue dans un plan transversal s'étendant à travers la couche couvrante de la coque en mousse métallique, (i) la couche couvrante définit une première zone en coupe transversale, (ii) la couche couvrante et le second segment d'âme définissent une deuxième zone en coupe transversale, et (iii) un rapport de la première zone en coupe transversale sur la deuxième zone en coupe transversale est plus grand que 0,30.

2. Prothèse orthopédique (10) selon la revendication 1, dans laquelle le rapport de la première zone en coupe transversale sur la deuxième zone en coupe transversale est de moins de 0,56, et de préférence est compris entre 0,35 et 0,47.

3. Prothèse orthopédique (10) selon la revendication 1, dans laquelle lorsque la prothèse orthopédique est vue dans un second plan transversal s'étendant à travers la gaine (52) de la coque en mousse métallique (50),
la gaine définit une troisième zone en coupe transversale,
la gaine et le premier segment d'âme définissent une quatrième zone en coupe transversale, et
un rapport de la troisième zone en coupe transversale sur la quatrième zone en coupe transversale est plus grand que 0,38.

4. Prothèse orthopédique (10) selon la revendication 3, dans laquelle le rapport de la troisième zone en coupe transversale sur la quatrième zone en coupe transversale est de moins de 0,70, et de préférence est compris entre 0,46 et 0,54.

5. Prothèse orthopédique (10) selon la revendication 3, dans laquelle le rapport de la troisième zone en coupe transversale sur la quatrième zone en coupe transversale est plus grand que le rapport de la première zone en coupe transversale sur la deuxième zone en coupe transversale.

6. Prothèse orthopédique (10) selon la revendication 1, dans laquelle :
la couche couvrante (58) de la coque en mousse métallique (50) s'étend de façon distale à partir d'une extrémité proximale (142) attachée à la gaine (52), et
le plan transversal s'étend à travers l'extrémité proximale (142) de la couche couvrante, et lorsque la prothèse orthopédique est vue dans le plan transversal :
(i) la couche couvrante a une première épaisseur, (ii) le second segment d'âme (66) a une seconde épaisseur, et (iii) un rapport de la première épaisseur sur la seconde épaisseur est plus grand que 0,46.

7. Prothèse orthopédique (10) selon la revendication 6, dans laquelle la première épaisseur est plus grande que 3 mm.

8. Prothèse orthopédique (10) selon la revendication 1, dans laquelle lorsque la prothèse orthopédique est vue dans le plan transversal, une surface médiale (38) du premier segment d'âme (64) est convexe et une surface latérale (40) du premier segment d'âme est convexe.

9. Prothèse orthopédique (10) selon la revendication 8, dans laquelle lorsque la prothèse orthopédique est vue dans le plan transversal, la surface médiale (38) du premier segment d'âme (64) est définie par un premier rayon, et la surface latérale (40) du premier segment d'âme (64) est définie par un second rayon qui est plus grand que le premier rayon.

10. Prothèse orthopédique (10) selon la revendication 1, dans laquelle :
le premier segment d'âme (64) de l'âme métallique (24) a une surface médiale (38) et une surface latérale (40) positionnée à l'opposé de la surface médiale du premier segment d'âme,
la gaine (52) a une surface médiale et une surface latérale positionnée à l'opposé de la surface médiale de la gaine, et
lorsque la prothèse orthopédique est vue dans le plan transversal, une première épaisseur (90) est définie entre un point le plus latéral (96) de la surface latérale de la gaine (52) et un point le plus latéral (94) de la surface latérale (70) du premier segment d'âme (64), (ii) une seconde épaisseur (92) est définie entre un point le plus médial (100) de la surface médiale (68) de la gaine (52) et un point le plus médial (100) de la surface médiale (68) de la première âme (64) et (iii) un rapport de la seconde épaisseur sur la première épaisseur est plus grand que 0,14.

11. Prothèse orthopédique (10) selon la revendication 1, dans laquelle la gaine (52) et la couche couvrante (58) de la coque en mousse métallique (50) ont un premier module d'élasticité et le premier segment d'âme (64) et le second segment d'âme (66) de l'âme métallique (32) ont un second module d'élasticité, le premier module d'élasticité étant approximativement d'un vingtième du second module d'élasticité.

12. Prothèse orthopédique (10) selon la revendication 1, comprenant :
un composant de tige fémorale allongé (14) ayant un axe longitudinal, le composant de tige comprenant :
la coque en mousse métallique (50) incluant la gaine (52), et
l'âme métallique (24) incluant (i) le col (26) positionné de façon proximale à la gaine, le col étant configuré pour recevoir un composant de tête fémorale, (ii) le premier segment d'âme (64) positionné dans la gaine, et (iii) le second segment d'âme (66) positionné de façon distale par rapport à la gaine,
dans laquelle (i) la coque en mousse en métallique inclut en outre la couche couvrante (58) s'étendant de façon distale à partir de la gaine, la couche couvrante n'engageant que la surface latérale (136) du second segment d'âme et définissant la première zone en coupe transversale en un premier point sur l'axe longitudinal, (ii) la couche couvrante et le second segment d'âme définissant la seconde zone en coupe transversale au niveau du premier point, et (iii) le rapport de la première zone en coupe transversale sur la seconde zone en coupe transversale est plus grand que 0,30.
